# EUROPÄISCHE PATENTSCHRIFT

(11) **EP 2 979 704 B1**
(45) Veröffentlichungstag und Bekanntmachung des Hinweises auf die Patenterteilung: **20.11.2019**
(21) Anmeldenummer: 14179106.1
(22) Anmeldetag: 30.07.2014
(51) Int. Cl.: A61K 38/10, A61P 37/02

(54) **Peptide des Desmoglein 3 bei Pemphigus vulgaris**
Peptides of desmoglein 3 in pemphigus vulgaris
Peptide de desmogléine 3 pour pemphigus vulgaire

(43) Veröffentlichungstag der Anmeldung: 03.02.2016
(73) Patentinhaber: PHILIPPS-UNIVERSITÄT MARBURG, 35037 Marburg (DE)
(72) Erfinder: Eming, Rüdiger, 35039 Marburg (DE); Hertl, Michael, 35039 Marburg (DE)
(74) Vertreter: Stumpf, Peter

(56) Entgegenhaltungen:
- WO-A1-2013/014247
- US-B1- 7 255 861
- YOKOYAMA T ET AL: "Antigen-independent development of Foxp3<+> regulatory T cells suppressing autoantibody production in experimental pemphigus vulgaris", INTERNATIONAL IMMUNOLOGY 2011 OXFORD UNIVERSITY PRESS GBR, Bd. 23, Nr. 6, 2011, Seiten 365-373, XP05147631, ISSN: 0953-8178
- TOMOAKI YOKOYAMA ET AL: "Immune dysregulation of pemphigus in humans and mice", JOURNAL OF DERMATOLOGY., Bd. 37, Nr. 3, 26. Februar 2010 (2010-02-26), Seiten 205-213, XP055147632, JP ISSN: 0385-2407, DOI: 10.1111/j.1346-8138.2009.00797.x

## Beschreibung

Die vorliegende Erfindung betrifft eine T-zellbasierte Immuntherapie von antikörpervermittelten Autoimmunerkrankungen bei Säugetieren, insbesondere bei Menschen, insbesondere die Verwendung von Peptiden, abgeleitet von humanem Desmoglein 3 (Dsg 3) zur Behandlung der Erkrankung Pemphigus vulgaris (PV). Durch die Applikation eines medizinischen Präparates, umfassend ein Peptid oder eine Kombination aus den Peptiden Seq-ID 1 - 5 wird eine Desmoglein 3-spezifische Immuntoleranz im Menschen ausgelöst.

Ebenso betrifft die vorliegende Erfindung eine T-zellbasierte Immuntherapie von antikörpervermittelten Autoimmunerkrankungen bei Hunden oder Pferden, insbesondere die Verwendung von Peptiden des artspezifischen Desmoglein 3 (Dsg 3) zur Behandlung der Erkrankung Pemphigus vulgaris (PV). Durch die Applikation eines oder einer Kombination aus den Peptiden Seq-ID 1 - 5 wird eine Desmoglein 3-spezifische Immuntoleranz im Hund oder Pferd ausgelöst.

Antikörper sind Proteine aus der Klasse der Globuline, die als Reaktion auf Antigene von Menschen oder Tieren gebildet werden und auch als Immunglobuline bezeichnet werden.

Epitope sind jene Bereiche eines Antigens, an denen ein Antikörper oder ein T-Zellrezeptor spezifisch binden.

Autoantikörper sind Antikörper, die als Reaktion auf körpereigene Antigene (Autoantigene) von Menschen oder Tieren gebildet werden. Autoantikörper sind charakteristisch für Autoimmunerkrankungen.

Das HLA-System (Human Leukocyte Antigen-System) beschreibt eine Gruppe von Genen, die für die Funktion des menschlichen Immunsystems wesentlich sind. Diese Gene sind beim Menschen auf dem Chromosom 6 lokalisiert und lassen sich in zwei Klassen einteilen: Klasse-I mit den Isotypen A, B und C sowie Klasse-II mit den Isotypen DR, DP und DQ. Diese Gene codieren die MHC-Proteinkomplexe, welche Antigene auf der Oberfläche von Körperzellen (beispielsweise Leukozyten) darstellen und zur Identifikation körpereigener Zellen dienen.

### Beschreibung des allgemeinen Gebietes der Erfindung

Die Erkrankung Pemphigus vulgaris (PV, Blasensucht, ICD-10-Klassifizierung L10.0) ist eine Autoimmunerkrankung der Haut und der hautnahen Schleimhäute und gehört zur Gruppe der blasenbildenden Autoimmundermatosen. Ursache der Erkrankung sind körpereigene Antikörper (IgG-Autoantikörper), welche gegen das körpereigene Protein Desmoglein 3 gerichtet sind. Das Desmoglein 3 fungiert somit als Autoantigen. Das Desmoglein 3 ist ein Proteinbestandteil des Desmosoms. Desmosomen sind Zellstrukturen in Epithelzellen, die einen mechanischen Zusammenhalt z.B. zwischen zwei epidermalen Keratinozyten der Haut bzw. Epithelzellen der Schleimhaut herstellen.

IgG-Autoantikörper gegen Desmoglein 3 induzieren verschiedene Effekte auf zellulärer und molekularer Ebene, die letztlich die interzelluläre Bindung der Desmogleine stören und bewirken einen Adhäsionsverlust epidermaler Keratinozyten, wodurch die klinischen Symptome der Erkrankung Pemphigus vulgaris, d.h. die Ausbildung von Blasen und Erosionen, verursacht werden.

Autoaggressive, d.h. gegen körpereigene Strukturen gerichtete CD4+ T-Zellen sind als Initiatoren der Autoimmunantwort bei der Erkrankung Pemphigus vulgaris wesentlich für die Aktivierung Autoantikörper-produzierender B-Zellen. CD4+ T-Zellen erkennen Epitope des Desmoglein 3 in Assoziation mit den Pemphigus vulgaris-assoziierten humanen Leukozytenantigenen (HLA)-Klasse-II-Allelen HLA-DRB1*04:02 und HLA-DQB1*05:03. Die Toleranzinduktion, d.h. die spezifische Hemmung autoreaktiver, d.h. gegen körpereigene Proteine gerichteter CD4+ T-Zellen setzt die Kenntnis der Desmoglein 3-Epitope voraus, die zur Stimulation autoaggressiver CD4+ T-Zellen führen.

### Stand der Technik

Die derzeit übliche Therapie der Erkrankung Pemphigus vulgaris besteht in der systemischen Verabreichung von hochdosierten Glukokortikoiden, beispielsweise Prednisolon, sowie von Immunsuppressiva, beispielsweise Azathioprin. Wesentlich dabei ist, dass es sich in der Regel um eine langfristige Therapie handelt, wodurch die potentiellen Nebenwirkungen der Glukokortikoide und der Immunsuppressiva bei zunehmender Krankheitsdauer den Therapieerfolg stark beeinträchtigen und zu erheblicher Komorbidität (Begleiterkrankungen) führen können. Diese beinhalten unter anderem Osteoporose, Diabetes mellitus, arterielle Hypertonie, Cushing-Syndrom, Hepatitis, Panzytopenie, opportunistische Infektionen und Thrombembolien.

Eine weitere Therapieoption ist die adjuvante, d.h. zusätzlich zur immunsuppressiven Medikation angewendete Immunadsorption, bei der versucht wird, die im Blut des Patienten zirkulierenden IgG-Antikörper und somit auch die Desmoglein 3-reaktiven Autoantikörper mit Hilfe einer Bindungssäule selektiv aus dem Körper des Patienten zu entfernen. Diese Therapie ist dadurch begrenzt, dass sie möglicherweise nur kurzfristig zur klinischen Remission führt und mit potentiellen Nebenwirkungen bei Anwendung eines zentralen Venenkatheters, z.B. Thrombembolien und bakteriellen Infektionen bis zur Sepsis verbunden ist.

Hochdosierte intravenöse Immunglobuline werden ebenfalls in der Therapie des Pemphigus vulgaris eingesetzt und führen über noch nicht vollständig geklärte Mechanismen zur Reduktion der zirkulierenden Desmoglein 3-spezifischen Autoantikörper. Mögliche Komplikationen sind Thrombosen, aseptische Meningitis und Leukopenie.

Seit dem 15.03.2019 ist der biotechnologisch hergestellte chimäre monoklonale Antikörper Rituximab, der gegen den Oberflächenrezeptor CD20 auf B-Lymphozyten gerichtet ist, vom Europäischen Parlament zur Behandlung des moderaten bis schweren Pemphigus vulgaris zugelassen. Bei Rituximab besteht das Risiko schwerer bakterieller und viraler Infektionen inklusive der potentiell lebensbedrohlichen progressiven, multifokalen Leukenzephalopathie (PML) als potentielle Komplikation.

Yokoyama T. et al. beschreiben in ihrer Veröffentlichung "Antigen-independent development of Foxp3+ regulatory T cells suppressing autoantibody production in experimental pemphigus vulgaris" (International Immunology 2011 Oxford University Press GBR, Bd. 23, Nr. 6, 2011, Seiten 365-373) die Entwicklung von Fox3+ regulatorischen T-Zellen (Tregs) zur Unterdrückung von Autoantikörpern in Pemphigus vulgaris. Die Autoren deuten auf die wichtige Rolle von Tregs bei der Kontrolle von Dsg3-reaktiven Lymphozyten hin.

Yokoyama T. und Amagei M. beschreiben in ihrer Veröffentlichung "Immune dysregulation of pemphigus in humans and mice" (Journal of Dermatology, Bd. 37, Nr. 3, 2010, Seiten 205-213) die immunologische Dysregulierung bei Pemphigus vulgaris in Menschen und Mäusen, wobei autoreaktive Lymphozyten von regulatorischen T-Zell-Untereinheiten wie CD4+, CD25+, Treg und IL-10 produzierenden Zellen (Tr1) unterdrückt werden.

In der Patentanmeldung WO 2013/014247 A1 beanspruchen die Erfinder Peptide als Teil eines Epitops von Antigenen aus einer Gruppe von Polypeptiden enthaltend unter anderem auch solche des Desmogleins 3 zur Behandlung von Autoimmunerkrankungen wie Pemphigus. Jedoch weisen sie nicht auf ein Peptid oder eine Kombination aus Peptiden hin, die eine Desmoglein 3-spezifische Immuntoleranz auslösen.

Die Patentschrift US 7 255 861 B1 offenbart isolierte Peptide in Zusammenhang mit Pemphigus vulgaris und multipler Sklerose, wobei es sich bei Pemphigus um Teile oder Epitope des humanen Proteins Desmoglein 3 handelt. Diese Peptide liegen jedoch nicht an einen Träger gekoppelt vor.

### Aufgabe

Die Aufgabe der vorliegenden Erfindung ist es, ein medizinisches Präparat zur Verfügung zu stellen, mit dem eine spezifischere und besser verträgliche Therapie der Erkrankung Pemphigus vulgaris erreicht wird.

### Lösung der Aufgabe

Die erfindungsgemäße Aufgabe wird gelöst durch die Ansprüche.

Die Erfindung besteht in der Bereitstellung eines medizinischen Präparats zur Verwendung bei Säugetieren, insbesondere bei Menschen, die an Pemphigus vulgaris (PV) erkrankt sind. Das medizinische Präparat umfasst mindestens ein oder eine Kombination aus den Peptiden Seq-ID 1 - 5 der immundominanten CD4+ T-Zellepitope des humanen Proteins Desmoglein 3, welche so an geeignete Träger gekoppelt sind, dass CD4+ T-Zellen aktiviert werden. Geeignete Träger sind Nanopartikel oder mononukleäre Zellen oder Erythrozyten oder dendritische Zellen. Diese Peptide induzieren eine Antigen-spezifische Immuntoleranz gegenüber Desmoglein 3 in Säugetieren, insbesondere in Menschen, wobei die Peptide Seq-ID 1 - 5 als nominelle Antigene wirken und eine Immuntoleranz im Patienten induzieren.

Die Erfindung besteht weiterhin in der Verwendung eines medizinischen Präparates bei der Behandlung der Krankheit Pemphigus vulgaris (PV) bei Menschen.

Die Erfindung besteht weiterhin in der Bereitstellung eines medizinischen Präparats zur Verwendung bei Hunden oder Pferden, die an Pemphigus vulgaris (PV) erkrankt sind. Das medizinische Präparat umfasst mindestens ein oder eine Kombination aus den Peptiden Seq-ID 1 - 5 der immundominanten CD4+ T-Zellepitope des caninen oder equinen Proteins Desmoglein 3, welche so an geeignete Träger gekoppelt sind, dass CD4+ T-Zellen aktiviert werden. Geeignete Träger sind Nanopartikel oder mononukleäre Zellen oder Erythrozyten oder dendritische Zellen. Diese Peptide induzieren eine Antigen-spezifische Immuntoleranz gegenüber Desmoglein 3 in Hunden oder Pferden, wobei die Peptide Seq-ID 1 - 5 als nominelle Antigene wirken und eine Immuntoleranz im Patienten induzieren.

Die Erfindung besteht weiterhin in der Verwendung eines medizinischen Präparates bei der Behandlung der Krankheit Pemphigus vulgaris (PV) bei Hunden oder Pferden.

Durch die Verabreichung eines medizinischen Präparates umfassend ein oder mehrere HLA-DRB1*04:02- und HLA-DQB1*05:03-bindende CD4+ T-Zell-Epitope des humanen Desmoglein 3 (Seq-ID 1-5) unter Immuntoleranz-erzeugenden Bedingungen bei Patienten mit Pemphigus vulgaris wird eine immunologische Toleranz gegenüber diesen CD4+ T-Zell-Epitopen des Desmoglein 3 (Seq-ID 1-5) induziert, so dass die Aktivierung autoaggressiver T-Zellen gehemmt wird. Daraus folgt eine mangelnde, T-zellabhängige Aktivierung von Desmoglein 3-spezifischen B-Zellen, so dass die Patienten mit Pemphigus vulgaris keine Autoantikörper gegen das Autoantigen Desmoglein 3 (Seq-ID 1-5) mehr bilden und die Erkrankung Pemphigus vulgaris damit zum Erliegen kommt. Es ist also für die Lösung der Aufgabe wesentlich, ein medizinisches Präparat bereitzustellen, welches die Funktion der Desmoglein 3-reaktiven CD4+ T-Zellen so beeinflusst, dass eine Immuntoleranz gegenüber Desmoglein 3 bei den Patienten mit Pemphigus vulgaris erzeugt wird.

Durch die Verabreichung eines oder mehrerer der HLA-DRB1*04:02- und HLA-DQB1*05:03-bindenden CD4+ T-Zell-Epitope des humanen Desmoglein 3 (Seq-ID 1-5) unter immuntoleranz-erzeugenden Bedingungen bei Patienten mit Pemphigus vulgaris entwickelt sich eine Immuntoleranz gegen das humane Desmoglein 3, wodurch der Erkrankung Pemphigus vulgaris die Grundlage entzogen wird.

Durch die Verabreichung eines oder mehrerer der HLA-DRB1*04:02- und HLA-DQB1*05:03-bindenden CD4+ T-Zell-Epitope des humanen Desmoglein 3 (Seq-ID 1-5) unter immuntoleranz-erzeugenden Bedingungen bei Patienten mit Pemphigus vulgaris verringert sich die Anzahl Desmoglein 3-spezifischer autoreaktiver CD4+ T-Zellen im peripheren Blut der Patienten mit Pemphigus vulgaris, sinken die Titer der Desmoglein 3-spezifischen Autoantikörper im Serum dieser Patienten. Somit bessern sich die klinischen Symptome und der Verbrauch der eingesetzten systemischen Glukokortikoide bzw. Immunsuppressiva verringert sich.

### Ausführungsbeispiele

In eigenen Versuchen wurden aus dem peripheren Blut von Pemphigus vulgaris-Patienten Desmoglein 3-spezifische CD4+ T-Zellklone isoliert und fünf CD4+ T-Zell-Epitope des humanen Desmoglein 3 in T-Zell-Proliferationsassays identifiziert (Seq-ID 1-5). Die fünf Epitope weisen definierte Bindungsmotive für das restringierende HLA-DRB1*04:02-Allel auf. Ebenfalls wurde nachgewiesen, dass die Erkennung dieser fünf CD4+ T-Zell-Epitope des Desmoglein 3 durch autoreaktive CD4+ T-Zellen auch durch ein zweites, mit Pemphigus vulgaris assoziiertes HLA-Klasse-II-Allel, nämlich HLA-DQB1*05:03 restringiert, d.h. beschränkt ist. Sowohl HLA-DRB1*04:02 als auch HLA-DQB1*05:03 weisen eine negative Ladung an den Positionen 70 und 71 der β1-Kette auf. Diese negative Ladung an der sog. p4-Bindungsstelle des HLA-Moleküls ist wesentlich für die Bindung der fünf CD4+ T-Zell-Epitope des humanen Desmoglein 3 (Seq-ID 1-5). Durch die Bindung der fünf CD4+ T-Zell-Epitope des humanen Desmoglein 3 (Seq-ID 1-5) an HLA-DRB1*04:02 und HLA-DQB1*05:03 werden die fünf CD4+ T-Zell-Epitope des humanen Desmoglein 3 (Seq-ID 1-5) autoaggressiven T-Zellen präsentiert und diese Desmoglein 3-spezifischen CD4+ T-Zellen werden aktiviert, was für die Produktion von Desmoglein 3-spezifischen Autoantikörpern und somit für die Entstehung des Pemphigus vulgaris essentiell ist.

### 1. Mausmodell zum Nachweis der Aktivierung von CD4+ T-Zellen durch die fünf CD4+ T-Zell-Epitope des humanen Desmoglein 3 (Seq-ID 1-5)

In einem HLA-DRB1*04:02-transgenen Mausmodell der Erkrankung Pemphigus vulgaris wurde gezeigt, dass die Aktivierung von CD4+ T-Zellen durch die fünf CD4+ T-Zell-Epitope des humanen Desmoglein 3 (Seq-ID 1-5) zur Induktion von pathogenen Desmoglein 3-spezifischen Autoantikörpern führt, welche letztendlich für die Erkrankung Pemphigus vulgaris ursächlich sind.

Nach der Immunisierung der transgenen Mäuse mit rekombinantem humanen Desmoglein 3 wurden CD4+ T-Zellen generiert, welche die Epitope des humanen Desmoglein 3 in Verbindung mit HLA-DRB1*04:02 erkennen. Die Immunisierung der HLA-DRB1*04:02-transgenen Mäuse mit den fünf CD4+ T-Zell-Epitopen des Desmoglein 3 (Seq-ID 1-5) führt zur Induktion pathogener Desmoglein 3-reaktiver IgG-Autoantikörper. Damit wurde erstmals in vivo bewiesen, dass Desmoglein 3-spezifische CD4+ T-Zellen, die immundominante Epitope des humanen Autoantigens im Kontext mit dem krankheitsassoziierten HLA-DRB1*04:02 erkennen, für die Produktion von Desmoglein 3-spezifischen Autoantikörpern und daher in der Pathogenese des Pemphigus vulgaris eine zentrale Rolle spielen.

### 2. Herstellung und Verabreichung eines medizinischen Präparates umfassend ein oder mehrere HLA-DRB1*04:02- und HLA-DQB1*05:03-bindende CD4+ T-Zell-Epitope des humanen Desmoglein 3 (Seq-ID 1-5) unter immuntoleranz-erzeugenden Bedingungen zur Behandlung von Patienten mit Pemphigus vulgaris

Für die Verabreichung eines oder mehrerer der HLA-DRB1*04:02- und HLA-DQB1*05:03-bindenden CD4+ T-Zell-Epitope des humanen Desmoglein 3 (Seq-ID 1-5) unter immuntoleranz-erzeugenden Bedingungen bei Patienten mit Pemphigus vulgaris stehen folgende Möglichkeiten zur Verfügung:

### 2.1 Medizinisches Präparat umfassend Konjugate der HLA-DRB1*04:02- und HLA-DQB1*05:03-bindenden CD4+ T-Zell-Epitope des Desmoglein 3 (Seq-ID 1-5) mit Nanopartikeln:

An geeignete Nanopartikel gekoppelte HLA-DRB1*04:02- und HLA-DQB1*05:03-bindende CD4+ T-Zell-Epitope des humanen Desmoglein 3 (Seq-ID 1-5) werden einem Patienten intravenös (i.v) verabreicht. Ein Beispiel für geeignete Nanopartikel sind mit Polymeren beschichtete Nanopartikel, an die Peptide gekoppelt sind. Diese Nanopartikel können beispielsweise superparamagnetische Eisenoxid-Nanokristalle sein, welche mit ⁵⁹Fe gelabelt sein können. Ein anderes Beispiel für geeignete Nanopartikel sind mit Polymeren beschichtete Nanopartikel in Form von Quantenpunkten. Diese Quantenpunkte können aus einem Kern mit Cadmiumselenid, einer ersten Hülle mit Cadmiumsulfid und einer zweiten Hülle mit Zinksulfid (CdSe/Cd/S/ZnS-core-shell-shell quantum dots) bestehen. Das für die Beschichtung der Nanopartikel geeignete Polymer kann ein amphiphiles Polymer sein. Ein Beispiel für ein amphiphiles Polymer ist Poly (Maleinsäureanhydrid-*alt*-1-Oktadecen) (Maleinsäure-Copoymer, Poly(maleic anhydride-*alt*-1-octadecene), maleated polymer).

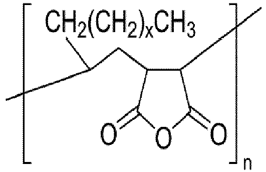

Die Koppelung der geeigneten Nanopartikel mit den Peptiden (HLA-DRB1*04:02- und HLA-DQB1*05:03-bindende CD4+ T-Zell-Epitope des humanen Desmoglein 3 (Seq-ID 1-5)) kann mit 1-Ethyl-3-(3-Dimethylaminopropyl)Carbodiimid (EDC) durchgeführt werden. Dafür können die geeigneten Nanopartikel in einer Konzentration von 6 µmol/l eingesetzt werden und EDC in einer Konzentration von 6 mmol/l. Zur Inkubation der Peptide (HLA-DRB1*04:02- und HLA-DQB1*05:03-bindende CD4+ T-Zell-Epitope des humanen Desmoglein 3 (Seq-ID 1-5)) kann EDC über Nacht in einem 100- bis 1000-fachen Überschuss eingesetzt werden.

### 2.2 Medizinisches Präparat, sowie dessen Herstellung und Verabreichung umfassend Konjugate der HLA-DRB1*04:02- und HLA-DQB1*05:03-bindenden CD4+ T-Zell-Epitope des humanen Desmoglein 3 (Seq-ID 1-5) mit mononukleären Zellen des peripheren Blutes oder mit Erythrozyten:

An patienteneigenen mononukleären Zellen oder Erythrozyten werden HLA-DRB1*04:02- und HLA-DQB1*05:03-bindende CD4+ T-Zell-Epitope des humanen Desmoglein 3 (Seq-ID 1-5) mit 1-Ethyl-3-(3-Dimethylaminopropyl)Carbodiimid (EDC) gekoppelt und dem betreffenden Patienten intravenös verabreicht. Die intravenöse Verabreichung kann eine Kurzinfusion sein.

HLA-DRB1*04:02- und HLA-DQB1*05:03-bindende CD4+ T-Zell-Epitope des humanen Desmoglein 3 (Seq-ID 1-5) werden mittels 1-Ethyl-3-(3-Dimethylaminopropyl)-Carbodiimid (EDC)-Behandlung an Zelloberflächen von patienteneigenen, (autologen) mononukleären Zellen oder Erythrozyten gekoppelt. Die Dosis der so präparierten, patienteneigenen mononukleären Zellen oder Erythrozyten für die Therapie des Pemphigus vulgaris liegt zwischen 1 x 10³ bis 3 x 10⁹ Zellen und kann im Lauf der Therapie variiert werden.

### 2.3 Medizinisches Präparat, sowie dessen Herstellung und Verabreichung umfassend Konjugate der HLA-DRB1*04:02- und HLA-DQB1*05:03-bindenden CD4+ T-Zell-Epitope des humanen Desmoglein 3 (Seq-ID 1-5) mit dendritischen Zellen:

Dendritische Zellen sind sehr effiziente, Antigen-präsentierende Immunzellen, die auf Grund ihrer immunmodulatorischen Eigenschaften im Bereich der immuntherapeutischen Forschung, insbesondere im Kontext von Tumor- und Autoimmunerkrankungen von großem Interesse sind. Auf Grund der besonderen Fähigkeit dendritischer Zellen, sowohl Immunantworten als auch immunologische Toleranz zu induzieren, nehmen sie eine zentrale Rolle in der Steuerung adaptiver Immunantworten ein. Zur Behandlung des Pemphigus vulgaris werden zunächst dendritische Zellen aus Blutmonozyten der Patienten generiert und diese dendritischen Zellen dann mit einem oder mehreren der HLA-DRB1*04:02- und HLA-DQB1*05:03-bindenden CD4+ T-Zell-Epitope des humanen Desmoglein 3 (Seq-ID 1-5) beladen. Die ex vivo mit den humanen Desmoglein 3-Epitopen (Seq-ID 1-5) beladenen dendritischen Zellen werden vergleichbar mit einer Vakzine den Pemphigus vulgaris-Patienten unter Immuntoleranz-erzeugenden Bedingungen wieder verabreicht, beispielsweise mittels einer intramuskulären oder subkutanen Injektion. Als Immuntoleranz-erzeugende Bedingungen wird verstanden, dass die dendritischen Zellen in einem möglichst unreifen Stadium gehalten werden.

### 3. Verabreichung eines medizinischen Präparates umfassend ein oder mehrere HLA-DRB1*04:02- und HLA-DQB1*05:03-bindende CD4+ T-Zell-Epitope des tierischen Desmoglein 3 unter Immuntoleranz-erzeugenden Bedingungen bei tierischen Patienten zur Behandlung von Pemphigus vulgaris

Die unter Punkt 2. beschriebenen medizinischen Präparate sind auch bei Tieren mit Pemphigus vulgaris, beispielsweise Hunde oder Pferde anwendbar. Das medizinische Präparat umfasst dabei anstatt der humanen Desmoglein 3-Epitope (Seq-ID 1-5) die jeweiligen caninen (bei Hunden) oder equinen (bei Pferden) Desmoglein 3-Epitope. Bei der Verwendung der unter Punkt 2. beschriebenen medizinischen Präparate bei anderen Tieren als Hund oder Pferd umfassen diese jeweils die artspezifischen Desmoglein 3-Epitope.

### 4. Charakterisierung immundominanter Epitope mittels Desmoglein 3-reaktiver T-Zellhybridome

Die funktionelle Charakterisierung der HLA-DRB1*04:02- und HLA-DQB1*05:03-bindenden CD4+ T-Zell-Epitope des humanen Desmoglein 3 (Seq-ID 1-5) wird exemplarisch für ein HLA-DRB1*4:02-restringiertes CD4+ T-Zell-Epitop des humanen Desmoglein 3 mit der Sequenz aa 505-521, SSPSVVVSARTLNNRYT (Seq-ID 6) gezeigt. Diese Charakterisierung wurde anhand von immortalisierten CD4+ T-Zellhybridomen durchgeführt. Die T-Zellhybridome sind nach Immunisierung von HLA-transgenen Mäusen mit rekombinantem, humanen Desmoglein 3-Protein generiert worden. Durch Fusion von Desmoglein 3-spezifischen CD4+ T-Zellen aus ableitenden regionären Lymphknoten Desmoglein 3-immunisierter HLA-transgener Mäuse mit der murinen Thymomzelllinie BW5147 entstehen Desmoglein 3-spezifische Hybridome, die nach antigenspezifischer Aktivierung Interleukin-2 (IL-2) sezernieren. Die Ergebnisse des Desmoglein 3-reaktiven T-Zellhybridoms, das spezifisch für das HLA-DRB1*04:02-restringierte CD4+ T-Zell-Epitop des Desmoglein 3 mit der Sequenz aa 505-521, SSPSVVVSARTLNNRYT (Seq-ID 6) ist, werden in Figur 1 gezeigt. Durch die Verwendung von verkürzten Varianten des nativen Desmoglein 3-Epitops mit der Sequenz aa 505-521, SSPSVVVSARTLNNRYT (Seq-ID 6) ist es möglich, den sogenannten Kernbereich (core epitope) des Epitops zu definieren; in diesem Fall hat das core epitope die Sequenz VVVSARTLN (core epitope der Seq-ID 6). Als core epitope wird der Bereich des Epitops bezeichnet, der mit der Bindungsgrube des HLA-DRB1*04:02-Molekül interagiert. Das core epitope ist der für die T-Zellaktivierung erforderliche Peptidbereich.

### Abbildungslegende

Figur 1 zeigt die Konzentration des T-Zellwachstumsfaktors Interleukin-2 (IL-2) im Kulturüberstand, welches nach antigenspezifischer Aktivierung von dem Desmoglein 3-reaktiven Zellhybridom sezerniert wurde. Dieses ist spezifisch für das HLA-DRB1*04:02-bindende CD4+ T-Zell-Epitop des humanen Desmoglein 3 mit der Sequenz aa 505-521, SSPSVVVSARTLNNRYT. Die Quantifizierung der Interleukin-2-Konzentrationen in den Kulturüberständen erfolgte mittels eines Interleukin-2-Enzyme-Linked-Immunosorbent-Assay (ELISA) und wird als Optical Density (OD) bei 450 nm Wellenlänge angegeben. Auf der y-Achse sind die verkürzten Varianten des Desmoglein 3-Epitops (aa 505-521, SSPSVVVSARTLNNRYT) aufgeführt.

### SEQUENCE LISTING

<110> Philipps-Universität Marburg
<120> Peptide des Desmoglein3 bei Pemphigus vulgaris
<130> TM819
<140> EP
   <141> 2014-07-21
<160> 6
<170> PatentIn version 3.5
<210> 1
   <211> 15
   <212> PRT
   <213> Homo sapiens
<220>
   <221> misc_feature
   <222> (1)..(15)
   <223> Dsg3 15-mer Peptid das an HLA-DRB1*04:02 und an HLA-DQB1*05:03 bindet
<400> 1
   Phe Gly Ile Phe Val Val Asp Lys Asn Thr Gly Asp Ile Asn Ile
   1               5                   10                  15
<210> 2
   <211> 15
   <212> PRT
   <213> homo sapiens
<220>
   <221> misc_feature
   <222> (1)..(15)
   <223> Dsg3 15-mer Peptid das an HLA-0RB1*04:02 und an HLA-DQB1*05:03 bindet
<400> 2
   Leu Asn Ser Lys Ile Ala Phe Lys Ile Val Ser Gln Glu Pro Ala
   1               5                   10                  15
<210> 3
   <211> 15
   <212> PRT
   <213> homo sapiens
<220>
   <221> misc_feature
   <222> (1)..(15)
   <223> Dsg3 15-mer Peptid das an HLA-DRB1*04:02 und an HLA-DQB1*05:03 bindet
<400> 3
   Thr Pro Met Phe Leu Leu Ser Arg Asn Thr Gly Glu Val Arg Thr
   1               5                   10                  15
<210> 4
   <211> 15
   <212> PRT
   <213> homo sapiens
<220>
   <221> misc_feature
   <222> (1)..(15)
   <223> Dsg3 15-mer Peptid das an HLA-DRB1*04:02 und an HLA-DQB1*05:03 bindet
<400> 4
   Cys Glu Cys Asn Ile Lys Val Lys Asp Val Asn Asp Asn Phe Pro
   1               5                   10                  15
<210> 5
   <211> 17
   <212> PRT
   <213> homo sapiens
<220>
   <221> misc_feature
   <222> (1)..(17)
   <223> Dsg3 17-mer Peptid das an HLA-DRB1*04:02 und an HLA-DQB1*05:03 bindet
<400> 5
   Ile Asn Val Arg Glu Gly Ile Ala Phe Arg Pro Ala Ser Lys Thr Phe
   1 5 10 15
   Thr
<210> 6
   <211> 17
   <212> PRT
   <213> homo sapiens
<220>
   <221> Core Epitop
   <222> (5)..(13)
   <223> Core Epitop, Bereich des Peptids der mit der Bindungsgrube des HLA-DRβ1*04:02-Moleküls interagiert; der für die T-zellaktivierung erforderliche Peptidbereich.
<220>
   <221> Peptid-T-zell Rezeptor-Bindungsstelle
   <222> (5)..(8)
   <223> Peptid-T-Zell Rezeptor-Bindungsstelle an Position 5 und Position 8
<220>
   <221> Peptid-HLA-DRB1*04:02-Bindungsstelle
   <222> (7)..(13)
   <223> Peptid-HLA-DRB1*04:02-Bindungsstelle an Position 7, Position 10, Position 12, Position 13
<400> 6

### Organization Applicant

Street : Biegenstr. 10
City : Marburg
State : Hessen
Country : Deutschland
PostalCode : 36037
PhoneNumber : 0641-94364-0
FaxNumber : 0641-94364-55
EmailAddress : patente@transmit.de
<110> OrganizationName : Philipps-Universität Marburg

### Application Project

<120> Title : Peptide des Desmoglein3 bei Pemphigus vulgaris
<130> AppFileReference : TM819
<140> CurrentAppNumber : EP
<141> CurrentFilingDate : 2014-07-21

### Sequence

<213> organismName : Homo sapiens
<400> Presequencestring :
   FGIFVVDKNT
   GDINI 15
<212> Type : PRT
<211> Length : 15 SequenceName : Dsg3 (97-111)-* SequenceDescription :

### Feature

Sequence: Dsg3 (97-111)*:
<221> FeatureKey : misc_feature
<222> LocationFrom : 1
<222> LocationTo : 15 Other Information : Dsg3 15-mer Peptid das an HLA-DRB1*04:02 und an HLA-DQB1*05:03 bindet CDSJoin : No

### Sequence

<213> organismName : homo sapiens
<400> Presequencestring :
   LNSKIAFKIV
   SQEPA 15
<212> Type : PRT
<211> Length : 15 SequenceName : Dsg3 (190-204)* SequenceDescription :

### Feature

Sequence: Dsg3 (190-204)*:
<221> FeatureKey : misc_feature
<222> LocationFrom : 1
<222> LocationTo : 15 Other Information : Dsg3 15-mer Peptid das an HLA-DRB1*04:02 und an HLA-DQB1*05:03 bindet CDSJoin : No

### Sequence

<213> organismName : homo sapiens
<400> PreSequenceString : TPMFLLSRNT GEVRT 15
<212> Type : PRT
<211> Length : 15 SequenceName : Dsg3 (206-220)* SequenceDescription :

### Feature

Sequence: Dsg3 (206-220)*:
<221> FeatureKey : misc_feature
<222> LocationFrom : 1
<222> LocationTo : 15 Other Information : Dsg3 15-mer Peptid das an HLA-DRB1*04:02 und an HLA-DQB1*05:03 bindet CDSJoin : No

### Sequence

<213> organismName : homo sapiens
<400> PreSequenceString :
   CECNIKVKDV
   NDNFP 15
<212> Type : PRT
<211> Length : 15 SequenceName : Dsg3(251-265)* SequenceDescription :

### Feature

sequence: Dsg3(251-265)*:
<221> FeatureKey : misc_feature
<222> LocationFrom : 1
<222> LocationTo : 15 Other Information : Dsg3 15-mer Peptid das an HLA-DRB1*04:02 und an HLA-DQB1*05:03 bindet CDSJoin : No

### sequence

<213> organismName : homo sapiens
<400> PreSequenceString : INVREGIAFR PASKTFT 17
<212> Type : PRT
<211> Length : 17 SequenceName : Dsg3 (375-391)* SequenceDescription :

### Feature

Sequence: Dsg3 (375-391)*:
<221> FeatureKey : misc_feature
<222> LocationFrom : 1
<222> LocationTo : 17 Other Information : Dsg3 17-mer Peptid das an HLA-DRB1*04:02 und an HLA-DQB1*05:03 bindet CDSJoin : No

### Sequence

<213> organismName : homo sapiens
<400> PreSequenceString : SSPSVVVSAR TLNNRYT 17
<212> Type : PRT
<211> Length : 17 SequenceName : Dsg3 (aa505-521) SequenceDescription :

### Feature

Sequence: Dsg3 (aa505-521):
<221> FeatureKey : Core Epitop
<222> LocationFrom : 5
<222> LocationTo : 13 other Information : Core Epitop, Bereich des Peptids der mit der Bindungsgrube des HLA-DRB1*04:02-Moleküls interagiert; der für die T-zellaktivierung erforderliche Peptidbereich. CDSJoin : No

### Feature

Sequence: Dsg3 (aa505-521):
<221> FeatureKey : Peptid-HLA-DRB1*04:02-Bindungsstelle
<222> LocationFrom : 7
<222> LocationTo : 13 other Information : Peptid-HLA-DRB1*04:02-Bindungsstelle an Position 7, Position 10, Position 12, Position 13 CDSJoin : No

### Feature

Sequence: Dsg3 (aa505-521):
<221> FeatureKey : Peptid-T-zell Rezeptor-Bindungsstelle
<222> LocationFrom : 5
<222> LocationTo : 8 other Information : Peptid-T-zell Rezeptor-Bindungsstelle an Position 5 und Position 8 CDSJoin : No

## Patentansprüche

1. Medizinisches Präparat zur Verwendung bei Säugetieren, insbesondere bei Menschen, die an Pemphigus vulgaris (PV) erkrankt sind, umfassend mindestens ein oder eine Kombination aus den Peptiden Seq-ID 1 - 5 der immundominanten CD4+ T-Zellepitope des humanen Proteins Desmoglein 3, welche so an geeignete Träger gekoppelt sind, dass CD4+ T-Zellen aktiviert werden, **dadurch gekennzeichnet, dass** die geeigneten Träger Nanopartikel oder mononukleäre Zellen oder Erythrozyten oder dendritische Zellen sind und dass diese Peptide eine Antigen-spezifische Immuntoleranz gegenüber Desmoglein 3 in Säugetieren, insbesondere in Menschen induzieren, wobei die Peptide Seq-ID 1 - 5 als nominelle Antigene wirken und eine Immuntoleranz im Patienten induzieren.

2. Medizinisches Präparat zur Verwendung bei Hunden oder Pferden, die an Pemphigus vulgaris (PV) erkrankt sind, beinhaltend mindestens ein oder eine Kombination aus den Peptiden Seq-ID 1 - 5 der immundominanten CD4+ T-Zellepitope des caninen oder equinen Proteins Desmoglein 3, welche an geeignete Träger gekoppelt sind, **dadurch gekennzeichnet, dass** die geeigneten Träger Nanopartikel oder mononukleäre Zellen oder Erythrozyten oder dendritische Zellen sind und dass diese Peptide eine Antigen-spezifische Immuntoleranz gegenüber Desmoglein 3 in Hunden oder Pferden induzieren, wobei die Peptide Seq-ID 1 - 5 als nominelle Antigene wirken und eine Immuntoleranz im Patienten induzieren.

3. Medizinisches Präparat nach Anspruch 1 zur Verwendung bei der Behandlung der Krankheit Pemphigus vulgaris (PV) bei Menschen.

4. Medizinisches Präparat nach Anspruch 2 zur Verwendung bei der Behandlung der Krankheit Pemphigus vulgaris (PV) bei Hunden oder Pferden.
**Anhängende Zeichnungen**

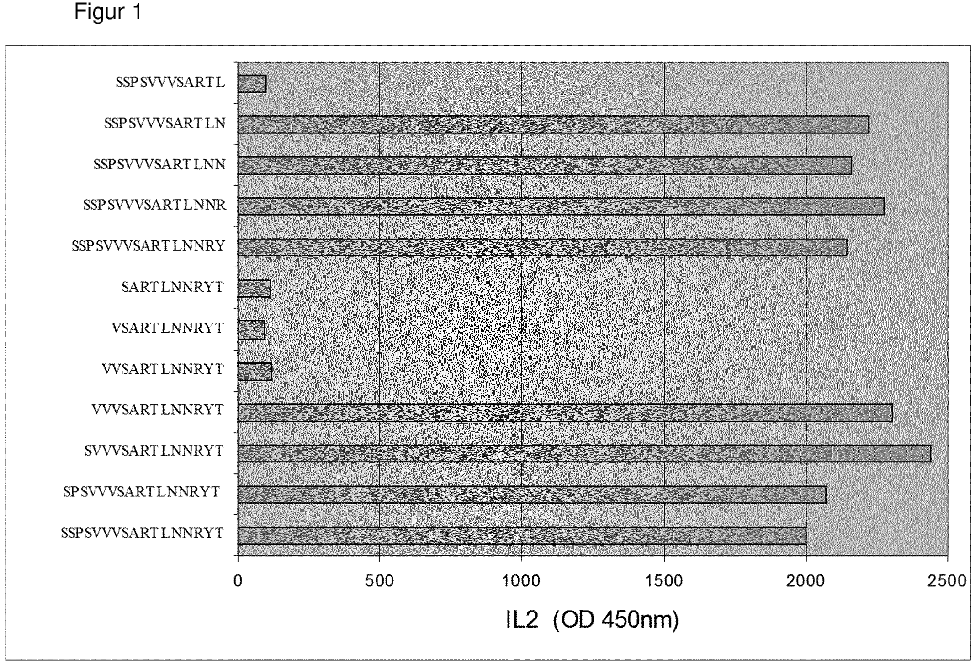

## Claims

1. A medical preparation for use in mammals, in particular in humans suffering from Pemphigus vulgaris (PV), comprising at least one or a combination of the peptides Seq-ID 1 - 5 of the immunodominant CD4+ T cell epitopes of the human protein desmoglein 3 coupled to suitable carriers such that CD4+ T cells are activated, **characterised in that** the suitable carriers are nanoparticles or mononuclear cells or erythrocytes or dendritic cells and **in that** these peptides induce an antigen-specific immunotolerance to desmoglein 3 in mammals, in particular in humans, wherein the peptides Seq-ID 1 - 5 act as nominal antigens and induce an immunotolerance in the patient.

2. A medical preparation for use in dogs or horses suffering from Pemphigus vulgaris (PV), comprising at least one or a combination of the peptides Seq-ID 1 - 5 of the immunodominant CD4+ T cell epitopes of the canine or equine protein desmoglein 3 coupled to suitable carriers, **characterized in that** the suitable carriers are nanoparticles or mononuclear cells or erythrocytes or dendritic cells and **in that** these peptides induce an antigen-specific immunotolerance to desmoglein 3 in dogs or horses, wherein the peptides Seq-ID 1 - 5 act as nominal antigens and induce an immunotolerance in the patient.

3. A medical preparation according to claim 1 for use in the treatment of the disease Pemphigus vulgaris (PV) in humans.

4. A medical preparation according to claim 2 for use in the treatment of the disease Pemphigus vulgaris (PV) in dogs or horses.

## Revendications

1. Préparation médicale destinée à être utilisée chez les mammifères, en particulier chez l'homme souffrant de Pemphigus vulgaris (PV), comprenant au moins un ou une combinaison des peptides Seq-ID 1 - 5 des épitopes immunodominants des cellules T CD4+ de la protéine humaine desmogléine 3 couplés à des supports appropriés de sorte que les cellules T CD4+ sont activées, **caractérisé en ce que** les supports appropriés sont des nanoparticules ou des cellules mononucléaires ou des érythrocytes ou des cellules dendritiques et **en ce que** ces peptides induisent une immunotolérance spécifique de l'antigène à la desmogléine 3 chez les mammifères, en particulier chez les humains, les peptides Seq-ID 1 - 5 agissant comme antigènes nominaux et induisant une immunotolérance chez le patient.

2. Préparation médicale destinée à être utilisée chez les chiens ou les chevaux souffrant de Pemphigus vulgaris (PV), comprenant au moins un ou une combinaison des peptides Seq-ID 1 - 5 des épitopes immunodominants des cellules T CD4+ de la protéine canine ou équine desmogléine 3 couplé à des supports appropriés, **caractérisée en ce que** les supports appropriés sont des nanoparticules ou des cellules mononucléaires ou des érythrocytes ou des cellules dendritiques et **en ce que** ces peptides induisent une immunotolérance spécifique de l'antigène à la desmogléine 3 chez le chien ou le cheval, les peptides Seq-ID 1 - 5 agissant comme antigènes nominaux et induisant une immunotolérance chez le patient.

3. Préparation médicale selon la revendication 1 pour utilisation dans le traitement de la maladie Pemphigus vulgaris (PV) chez l'homme.

4. Préparation médicale selon la revendication 2 pour utilisation dans le traitement de la maladie Pemphigus vulgaris (PV) chez le chien ou le cheval.
